# FASCICULE DE BREVET EUROPEEN

(11) **EP 0 828 837 B1**
(45) Date de publication et mention de la délivrance du brevet: **09.01.2008**
(21) Numéro de dépôt: 96920888.3
(22) Date de dépôt: 03.06.1996
(51) Int. Cl.: C12N 15/82, C12N 5/10, A01H 5/00

(54) **GENE CHIMERE COMPRENANT UNE SEQUENCE ADN D'UN GENE DE L'HYDROXY-PHENYL PYRUVATE DIOXYGENASE ET OBTENTION DE PLANTES CONTENANT UN GENE DE L'HYDROXY-PHENYL PYRUVATE DIOXYGENASE, TOLERANTES A CERTAINS HERBICIDES**
CHIMAERES GEN, DAS EIN HYDROXY-PHENYLPYRUVAT DIOXYGENASE-GEN KODIERENDE DNA SEQUENZ ENTHELT, UND HERSTELLUNG DIESE ENTHALTENDE HERBIZID-RESISTENZEN PFLANZEN
CHIMERIC GENE COMPRISING A DNA SEQUENCE OF A GENE OF HYDROXY-PHENYL PYRUVATE DIOXYGENASE AND PRODUCTION OF PLANTS CONTAINING A GENE OF HYDROXY-PHENYL PYRUVATE DIOXYGENASE AND WHICH ARE TOLERANT TO CERTAIN HERBICIDES

(30) Priorité: 02.06.1995 FR 9506800; 10.11.1995 FR 9513570; 07.05.1996 FR 9605944
(43) Date de publication de la demande: 18.03.1998
(73) Titulaire: Bayer CropScience S.A., 69009 Lyon (FR)
(72) Inventeur: SAILLAND, Alain, F-69009 Lyon (FR); ROLLAND, Anne, F-69009 Lyon (FR); MATRINGE, Michel, F-69009 Lyon (FR); PALLETT, Ken, Essex CM5 0HW (GB)
(86) Numéro de dépôt international: PCT/FR1996/000831
(87) Numéro de publication internationale: WO 1996/038567

(56) Documents cités:
- EP-A- 0 507 698
- EP-A- 0 508 909
- EP-A- 0 614 970
- EP-A- 0 652 286
- JOURNAL OF BACTERIOLOGY 176 (17). 1994. 5312-5319., XP002028042 DENOYA C. D., ET AL.: "A Streptomyces avermitilis gene encoding a 4-hydroxyphenylpyruvic acid dioxygenase-like protein that directs the production of homogentisic acid and an ochronotic pigment in Escherichia coli."
- EUR J BIOCHEM 205 (2). 1992. 459-466, XP002028045 RUETSCHI U., ET AL.: "CHARACTERIZATION OF 4 HYDROXYPHENYLPYRUVATE DIOXYGENASE PRIMARY STRUCTURE OF THE PSEUDOMONAS ENZYME."
- INFECT. IMMUN. (1994), 62(3), 1109-17, XP002028047 WINTERMEYER, EVA ET AL: "Sequence determination and mutational analysis of the lly locus of Legionella pneumophila"
- THE JOURNAL OF BIOLOGICAL CHEMISTRY, vol. 267, no. 34, 5 Décembre 1992, pages 24235-24240, XP002028043 ENDO, F., ET AL.: "Primary structure deduced from complementary DNA sequence and expression in cultured cells of mammalian 4-hydroxyphenylpyruvic acid dioxygenase"
- PHOTOSYNTHESIS: FROM LIGHT TO BIOSPHERE. VOLUME V. PROCEEDINGS OF THE XTH INTERNATIONAL PHOTOSYNTHESIS CONGRESS, MONTPELLIER, FRANCE, 20-25 AUGUST, 1995, (1995) PP. 285-288. 7 REF. PUBLISHER: KLUWER ACADEMIC PUBLISHERS. DORDRECHT, XP000646348 LENNE, C. ET AL: "Localization and partial purification of p- hydroxyphenylpyruvate dioxygenase from cultured carrot cells"
- MOLECULAR CLONING A LABORATORY MANUAL SECOND EDITION., pages 14.7-14.8, XP002028046 SAMBROOK, J., ET AL.: "Generation of probes specific for uncloned genes by selective amplification of particular segments of cDNA"
- EMBL SEQUENCE DATABASE, REL. 40, 16-JUN-1994, ACCESSION NO. T20952, XP002028637 NEWMAN, T., ET AL.: "2960 Arabidopsis thaliana cDNA clone 91B13T7"
- EMBL SEQUENCE DATABASE, REL. 47, 8-MAR-1996, ACCESSION NO. N65764, XP002029449 NEWMAN, T., ET AL.: "20804 Araabidopsis thaliana cDNA clone 231K20T7"
- GENE (AMSTERDAM) 161 (1). 1995. 107-111., XP002028636 WYCKOFF E E ET AL: "Cloning and expression of a gene encoding a T-cell reactive protein from Coccidioides immitis: Homology to 4--hydroxyphenylpyruvate dioxygenase and the mammalian F antigen."
- GENOMICS, vol. 23, no. 3, 1 Octobre 1994, pages 534-539, XP000561826 HISATAKA AWATA ET AL: "STRUCTURE OF THE HUMAN 4-HYDROXYPHENYLPYRUVIC ACID DIOXYGENASE GENE (HPD)"
- FEMS MICROBIOLOGY LETTERS 124 (2). 1994. 179-184., XP002028048 RUZAFA C., ET AL.: "The protein encoded by the Shewanella colwelliana melA gene is a p-hydroxyphenylpyruvate dioxygenase"
- GENE, vol. 109, pages 131-136, XP002028044 FUQUA, W.C., ET AL.: "Characterization of melA: a gene encoding melanin biosynthesis from the marine bacterium Shewanella colwelliana"
- FEBS LETTERS, vol. 318, no. 2, AMSTERDAM NL, pages 162-166, XP002028049 SCHULZ, A., ET AL.: "SC-0051, a 2-benzoyl-cyclohexane-1,3-dione bleaching herbicide, is a potent inhibitor of the enzyme p-hydroxyphenylpyruvate dioxygenase"
- 41ST HUNGARIAN PLANT PROTECTION DAYS, BUDAPEST, HUNGARY, FEBRUARY 21-22, 1995. PESTICIDE SCIENCE 45 (3). 1995. 286-287., XP000547268 BARTA I C ET AL: "Benzoylcyclohexanedione herbicides are strong inhibitors of purified p- hydroxyphenylpyruvic acid dioxygenase of maize."
- PLANT PHYSIOLOGY, (1994) VOL. 106, NO. 4, PP. 1429-1433., XP002028050 SECOR, J.: "Inhibition of barnyardgrass 4- hydroxyphenylpyruvate dioxygenase by sulcotrione"
- THE PLANT JOURNAL, vol. 6, no. 4, 1994, pages 481-489, XP002017203 MISAWA, N., ET AL.: "Expression of an Erwinia phytoene desaturase gene not only confers multiple resistance to herbicides interfering with carotenoid biosynthesis but also alters xanthophyll metabolism in transgenic plants"

## Description

La présente invention concerne un gène chimère contenant un gène de l'hydroxy-phényl pyruvate dioxygénase (HPPD), comme séquence codante et son utilisation pour l'obtention de plantes résistantes à certains herbicides.

On connait certains herbicides tels que les isoxazoles décrites notamment dans les demandes de brevets français 95 06800 and 95 13570 et notamment l'isoxaflutole, herbicide sélectif du maïs, les dicétonitriles tels que ceux décrits dans les demandes européennes 0 496 630, 0 496 631, en particulier la 2-cyano-3-cyclopropyl-1-(2-SO₂ CH₃-4-CF₃ phényl) propane-1,3-dione et la 2-cyano-3-cyclopropyl-1-(2-SO CH₃-2,3 Cl₂ phényl) propane-1, 3-dione, les tricétones décrites dans les demandes européennes 0 625 505 et 0 625 508, en particulier la sulcotrione. Cependant aucun gène de tolérance à de tels herbicides n' a été décrit.

L'hydroxy-phényl pyruvate dioxygénase est une enzyme qui catalyse la réaction de transformation du para-hydroxy-phényl-pyruvate en homogentisate.

Par ailleurs, la séquence en acides aminés de l'hydroxy-phényl pyruvate dioxygénase issue de *Pseudomonas sp.* P.J. 874 a été décrite, sans qu'il y ait une description de son rôle dans la tolérance des plantes aux herbicides (Rüetschi et col: Eur. J. Biochem. 205, 459-466, 1992). Ce document ne donne pas de description du gène codant pour cette protéine.

Il a maintenant été découvert la séquence d'un gène de ce type et qu'une telle séquence pouvait, une fois incorporée dans des cellules végétales, fournir une surexpression ou une activation de l'HPPD dans les plantes conférant à ces dernières une tolérance intéressante à certains herbicides récents, tels que ceux de la famille des isoxazoles ou de celle des tricétones.

Plus particulièrement cette séquence peut être d'origine bactérienne, telle que notamment le genre *Pseudomonas* ou encore d'origine végétale, telle que notamment de plante monocotylédone ou dicotylédone, notamment *d'Arabidopsis* ou d'ombellifères comme par exemple la carotte *(Daucus carotta).* Elle peut être native ou sauvage ou éventuellement mutée tout en gardant fondamentalement une propriété de tolérance herbicide contre les inhibiteurs de l'HPPD, tels que les herbicides de la famille des isoxazoles ou de celle des tricétones.

Les gènes codant pour une HPPD peuvent être isolés selon le procédé suivant:
- on choisit, comme amorces, quelques oligonucléotides issus de la séquence en acides aminés d'une HPPD,
- à partir de ces amorces, on synthétise des fragments d'amplification par PCR
- on isole le gène par création et criblage d'une banque génomique et
- on clone le gène.

De préférence on utilise des amorces issues de la séquence de l'HPPD d'une bactérie du genre *Pseudomonas.* De manière particulièrement préférée, elles sont issues de *Pseudomonas fluorescens.*

L'invention a encore pour objet l'utilisation d'un gène codant pour l'HPPD dans un procédé pour la transformation des plantes, comme gène marqueur ou comme séquence codante permettant de conférer à la plante une toléranceà certains herbicides. Il peut également être utilisé en association avec d'autres gènes marqueurs et/ou séquence codante pour une propriété agronomique.

Le gène codant peut être de toute origine, natif ou sauvage ou éventuellement muté tout en gardant fondamentalement une propriété de tolérance herbicide contre les inhibiteurs de l'HPPD, tels que les herbicides de la famille des isoxazoles ou de celle des tricétones. Comme séquence codante on peut notamment utiliser celle selon l'invention telle que décrite ci-dessus.

La transformation des cellules végétales peut être obtenue par tout moyen connu approprié. Une série de méthodes consiste à bombarder des cellules ou des protoplates avec des particules auxquelles sont accrochées les séquences d'ADN.

Une autre série de méthodes consiste à utiliser comme moyen de transfert dans la plante d'un gène chimère inséré dans un plasmide Ti d'*Agrobacterium tumefaciens* ou Ri *d'Agrobacterium rhizogenes.*

La présente invention a pour objet un gène chimère comprenant, dans le sens de la transcription, au moins une séquence de régulation promotrice, une séquence de peptide de transit d'un gène végétal codant pour une enzyme à localisation plastidiale entre la séquence le régulation promotrice et la séquence codante une séquence codante hétérologue qui exprime l'hxdroxy-phényl pyruvate dioxygénase et au moins une séquence de régulation terminatrice ou de polyadénylation.

Comme séquence de régulation promotrice on peut utiliser toute séquence promotrice d'un gène s'exprimant naturellement dans les plantes en particulier un promoteur d'origine bactérienne, virale ou végétale tel que, par exemple, celui d'un gène de la petite sous-unité de ribulose-biscarboxylase (RuBisCO) ou de celui d'un gène de l'α tubuline (Demande européennne EP n° 0 652 286), ou encore d'un gène de virus de plante tel que, par exemple, celui de la mosaïque du choux fleur (CAMV 19S ou 35S), mais tout promoteur convenable connu peut être utilisé. De préférence on a recours à une séquence de régulation promotrice qui favorise la surexpression de la séquence codante, tel que par exemple, celle comprenant au moins un promoteur d'histone tel que décrit dans la demande européenne EP 0507698.

Selon l'invention, on peut également utiliser, en association avec la séquence de régulation promotrice, d'autres séquences de régulation, qui sont situées entre le promoteur et la séquence codante, telles que des activateurs de trancription "enhancer", comme par exemple l'activateur de translation du virus etch du tabac (TEV) décrit dans la demande WO87/07644. Les peptides de transit sont soit simples, soit doubles, et dans ce cas éventuellement séparés par une séquence intermédiaire, c'est à dire comprenant, dans le sens de la transcription, une séquence codant pour un peptide de transit d'un gène végétal codant pour une enzyme à localisation plastidiale, une partie de séquence de la partie mature N terminale d'un gène-végétal codant pour une enzyme à localisation plastidiale, puis une séquence codant pour un second peptide de transit d'un gène végétal codant pour une enzyme à localisation plastidiale.constituée d'une partie de séquence de la partie mature N terminale d'un gène végétal codant pour une enzyme à localisation plastidiale, tels que décrit dans la demande européenne n° 0 508 909.

Comme séquence de régulation terminatrice ou de polyadénylation, on peut utiliser toute séquence correspondante d'origine bactérienne, comme par exemple le terminateur nos *d'Agrobacterium tumefaciens,* ou encore d'origine végétale, comme par exemple un terminateur d'histone tel que décrit dans la demande européenne EP n° 0 633 317.

La présente invention a encore pour objet les cellules végétales, de plantes monocotylédones ou dicotylédones, notamment des cultures, transformées selon l'un des procédés décrits ci-dessus et contenant dans leur génome une quantité efficace d'un gène exprimant l'hydroxy-phényl pyruvate dioxygénase (HPPD). On a observé que des plantes transformées de cette façon présentent une toléranceimportante à certains herbicides récents tels que lese isoxazoles décrites notamment dans les demandes de brevets français 9506800 and 95 13570 et notamment du 4-[4-CF3-2-(méthylsulfoyl) benzoyl]-5-cyclopropyl isoxazole, et notamment l'isoxaflutole, herbicide sélectif du maïs, , les dicétonitriles tels que ceux décrits dans les demandes européennes 0 496 630, 0 496 631, en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones décrites dans les demandes européennes 0 625 505 et 0 625 508, en particulier la sulcotrione.

L'invention a enfin pour objet un procédé de désherbage de plantes, notamment de cultures, à l'aide d'un herbicide de ce type, caractérisé en ce qu'on applique cet herbicide sur des plantes transformées selon l'invention, tant en présemis, en prélevée qu'en postlevée de la culture.

L'invention a encore pour objet l'utilisation du gène HPPD comme gène marqueur au cours du cycle "transformation-régénération" d'une espèce végétale et sélection sur l'herbicide ci-dessus

Les différents aspects de l'invention seront mieux compris à l'aide des exemples expérimentaux ci-dessous.

### Exemple 1: Isolement du gène de l'HPPD de P. fluorescens A32.

A partir de la séquence en acides aminés de l'HPPD de *Pseudomonas sp.* P.J. 874 ( publié par Rüetschi U. et al. 1992. Eur. J. Biochem. 205: 459-466), on déduit la séquence de différents oligonucléotides pour amplifier par PCR une partie de la séquence codante de l'HPPD de *P. fluorescens* A32 (isolée par McKellar, R.C. 1982. J. Appl Bacteriol. 53:305-316). Un fragment d'amplification du gène de cette HPPD a été utilisé pour cribler une banque génomique partielle de *P. fluorescens* A32 et ainsi isoler le gène codant pour cette enzyme.

### A) Préparation de l'ADN génomique de P. fluorescens A32.

La bactérie a été cultivée dans 40 ml de milieu minnimum M63 (KH₂PO₄ 13,6g/l, (NH₄)₂SO₄ 2g/l, MgSO₄ 0,2g/l, FeSO₄ 0,005 g/l pH7 plus L-tyrosine 10mM comme seule source de carbone) à 28°C pendant 48 heures.

Après lavage, les cellules sont reprises dans 1 ml de tampon de lyse (tris HCl 100 mM pH 8,3, NaCl 1,4 M et EDTA 10 mM) et incubées 10 minutes à 65°C. Après un traitement au phénol/chloroforme (24/1) et un traitement au chloroforme, les acides nucléiques sont précipités par addition d'un volume d'isopropanol puis repris dans 300 µl d'eau stérile et traités à la RNAse 10 µg/ml final. L'ADN est de nouveau traité au phénol/chloroforme, chloroforme et reprécipité par addition de 1/10 de volume d'acétate de sodium 3M pH5 et 2 volumes d'éthanol. L'ADN est ensuite repris dans de l'eau stérile et dosé.

### B) Choix des oligonucléotides et synthèses.

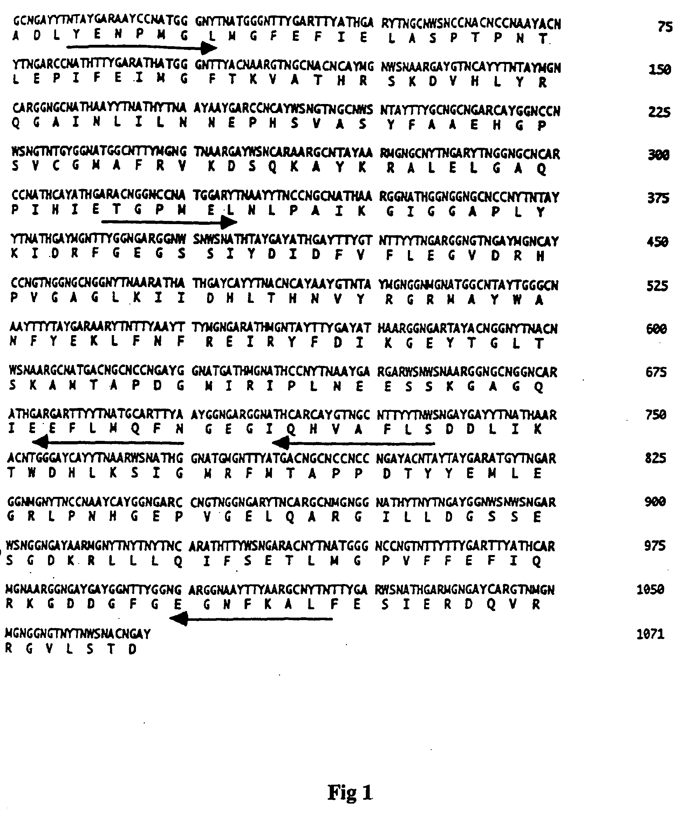

A partir de la séquence en acides aminés de l'HPPD de *Pseudomonas sp.* P.J. 874 on choisit cinq oligonucléotides, deux dirigés dans le sens NH₂ terminal de la protéine vers le COOH terminal de la protéine et trois dirigés dans le sens inverse (voir figure 1). Le choix a été dicté par les deux règles suivantes:
- une extrémité 3' de l'oligonucléotide stable, c'est à dire au moins deux bases sans ambiguité.
- une dégénérescence la plus faible possible.

Les oligonucléotides choisis ont les séquences suivantes:
P1: 5TA(C/T)GA(G/A)AA(C/T)CCIATGGG3'
P2: 5'GA(G/A)ACIGGICCIATGGA3'
P3: 5'AA(C/T)TGCATIA(G/A)(G/A)AA(C/T)TC(C/T)TC3'
P4: 5'AAIGCIAC(G/A)TG(C/T)TG(T/G/A)ATICC3'
P5: 5'GC(C/T)TT(A/G)AA(A/G)TTICC(C/T)TCICC3'

Ils ont été synthétisés sur le synthétiseur "Cyclone plus DNA Synthesizer" de marque MILLPORE.

Avec ces cinq oligonucléotides par PCR les fragments d'amplification que l'on doit obtenir théoriquement d'après la séquence SEQ ID N° 1 ont les tailles suivantes:
avec les amorces P1 et P3 ------> environ 690 bp
avec les amorces P1 et P4 ------> environ 720 bp
avec les amorces P1 et P5 ------> environ 1000 bp
avec les amorces P2 et P3 ------> environ 390 bp
avec les amorces P2 et P4 ------> environ 420 bp
avec les amorces P2 et P5 ------> environ 700 bp

### C) Amplification d'une partie codante de l'HPPD de P. fluorescens A32.

Les amplifications ont été faites sur un appareil PCR PERKIN ELMER 9600 et avec la Taq polymérase PERKIN ELMER avec son tampon dans les conditions standards, c'est à dire pour 50µl de réaction il y a les dNTP à 200µM, les primers à 20µM, la Taq polymérase 2,5 unités et l'ADN de *P. fluorescens* A32 2,5 µg.

Le programme d'amplification utilisé est, 5 min à 95°C puis 35 cycles <45 sec 95°C, 45 sec 49°C, 1 min 72°C> suivis de 5 min à 72°C.

Dans ces conditions, tous les fragments d'amplification obtenus ont une taille compatible avec les tailles théoriques données au-dessus, ce qui est une bonne indication de la spécificité des amplifications.

Les fragments d'amplifications obtenus avec les jeux d'amorces P1/P4, P1/P5 et P2/P4 sont ligués dans pBSII SK(-) après digestion de ce plasmide par Eco RV et traitement à la terminal transférase en présence de ddTTP comme décrit dans HOLTON T.A. and GRAHAM M.W. 1991. N.A.R. vol 19, n°5 p1156.

Un clone de chacun des trois types est séquencé partiellement; ceci permet de confirmer qu'on a bien amplifié dans les trois cas une partie de la région codante de l'HPPD de *P. fluorescens* A32. Le fragment P1/P4 est retenu comme sonde pour cribler une banque génomique partielle de *P. fluorescens* A32 et isoler le gène complet de l'HPPD.

### D) Isolement du gène.

Par Southern on montre qu'un fragment de 7 Kbp après digestion de l'ADN de *P*. *fluorescens* A32 par l'enzyme de restriction BamHI s'hybride avec la sonde HPPD P1/P4. On a donc fait digérer 400µg d'ADN de *P. fluorescens* A32 par l'enzyme de restriction BamHI et purifier sur gel d'agarose les fragments d'ADN faisant environ 7Kbp.

Ces fragments sont ligués dans pBSII SK(-), lui-même digéré par Bam HI et déphosphorylé par traitement à la phosphatase alcaline. Après transformation dans *E*. *coli* DH10b, la banque génomique partielle est criblée avec la sonde HPPD P1/P4.

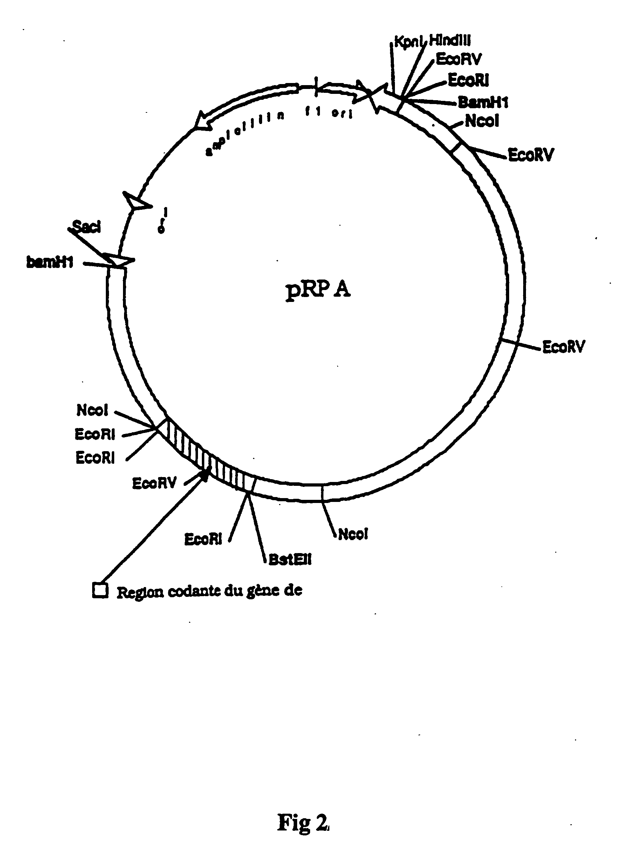

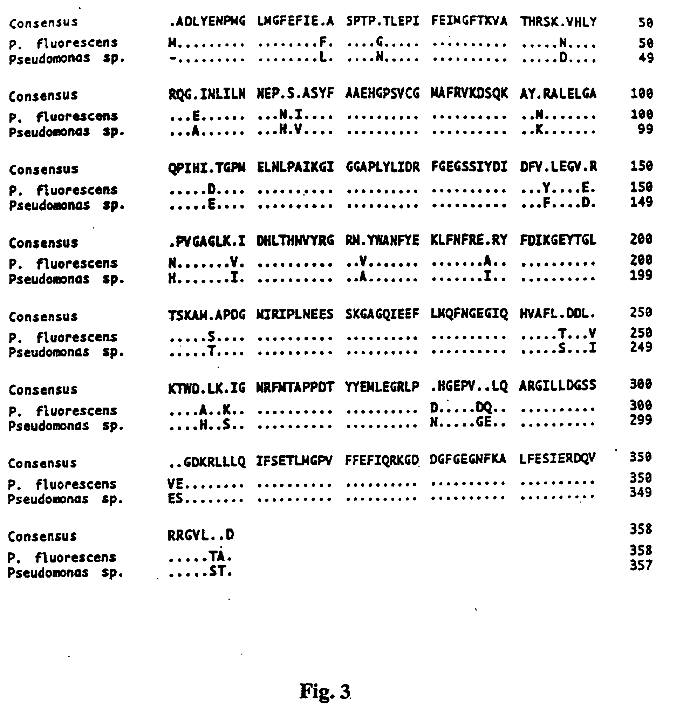

Un clone positif a été isolé et appelé pRP A. Sa carte simplifiée est donnée figure 2. Sur cette carte est indiqué la position de la partie codante du gène HPPD. Elle est composée de 1077 nucléotides qui codent pour 358 acides aminés (voir SEQ ID N° 1). L'HPPD de *P. fluorescens* A32 présente une bonne homologie en acides aminés avec celle de *Pseudomonas* sp. strain P.J. 874, il y a en effet 92% d'identité entre ces deux protéines ( voir figure 3).

### Exemple 2: Construction de deux gènes chimères.

Pour conférer la tolérancede plantes aux herbicides inhibant l'HPPD, on construit deux gènes chimères:

Le premier consiste à mettre la partie codante du gène de l'HPPD de *P. fluorescens* A32 sous le controle du promoteur double histone (Demande de Brevet européen N° 0 507 698) suivi du Tobacco etch virus translational enhancer (TEV) (pRTL-GUS (Carrington and Freed, 1990; J. Virol. 64: 1590-1597)) avec le terminateur du gène de la nopaline synthase. L'HPPD sera alors localisée dans le cytoplasme.

Le deuxième sera identique au premier, à ceci près qu'entre l'activateur de translation TEV et la partie codante de l'HPPD, on intercale le peptide de transit optimisé (OTP) (Demande européennne EP n° 0 508 909). L'HPPD sera alors localisée dans le chloroplaste.

### A) Construction du vecteur pRPA-RD-153:

- pRPA-RD-11 Un dérivé de pBS-II SK(-) (Stratagene catalog #212206) contenant le site de polyadenylation de la nopaline synthase (NOS polyA) (Demande européennne EP n° 0 652 286) est cloné entre les sites *KpnI* et *SalI.* Le site *KpnI* est transformé en un site NotI par traitement avec la T4 ADN polymerase I en présence de 150 µM de deoxynucleotide triphoshates puis ligation avec un linker NotI (Stratagene catalog #1029). Ainsi on obtient une cassette de clonage NOS polyA .
- pRPA-RD-127: Un dérivé de pRPA-BL-466 (Demande européennne EP n° 0 337 899) cloné dans pRPA-RD-11 créant une cassette d'expression du gène *oxy* et contenant le promoteur de la petite sous unité de la ribulose-biscarboxylase:

### " promoter (SSU) - oxy gène - NOS polyA"

Pour créer ce plasmide, pRPA-BL-488 a été digéré avec XbaI et HindIII pour isoler un fragment de 1.9 kbp contenant le promoteur SSU et le gène *oxy* qui a été ligué dans le plasmide pRPA-RD-11 digéré avec des enzymes compatibles.
- pRPA-RD-132: C'est un dérivé de pRPA-BL-488 (Demande européennne EP n° 0 507 698) cloné dans pRPA-RD-127 avec création d'une cassette d'expression du gène *oxy* avec le promoteur double histone:

### " promoteur double histone - oxy gene - NOS polyA "

Pour fabriquer ce plasmide, pRPA-BL-466 est digéré par HindIII, traité par la Klenow puis redigéré avec NcoI. Le fragment de 1.35 kbp purifié contenant le promoteur double histone H3A748 est ligué avec le plasmide pRPA-RD-127 qui avait été digéré par XbaI, traité Klenow et redigéré par NcoI.
- pRPA-RD-153: C'est un derivé de pRPA-RD-132 contenant l'activateur de translation du virus etch du tabac (TEV). pRTL-GUS (Carrington and Freed, 1990; J. Virol. 64: 1590-1597) est digéré avec *NcoI* et *EcoRI* et le fragment de 150 bp est ligué dans pRPA-RD-132 digéré avec les mêmes enzymes. Donc on a créé une cassette d'expression contenant le promoteur:

### "double histone promoter - TEV -oxy gene - NOS polyA"

### B) Construction du vecteur pRPA-RD-185:

pUC19/GECA: Un dérivé de pUC-19 (Gibco catalog #15364-011) contenant de nombreux sites de clonage. pUC-19 est digéré avec *EcoRI* et ligué avec l'oligonucleotide linker 1:

Le clone sélectionné contient un site *EcoRI* suivi du polylinker qui contient les sites suivants: *EcoRI, ApaI, AvrII, PmeI, SfiI, SacI, KpnI, SmaI, BamHI, Xbal, SalI, PstI, SphI* et *HindIII.*

pRPA-RD-185: c'est un dérivé de pUC19/GECA contenant un polylinker modifié. pUC19/GECA est digéré par HindIII et ligué avec l'oligonucleotide linker 2:

Le clone sélectionné contient un site *HindIII* site au milieu du polylinker qui contient maintenant les sites suivants: *EcoRI, ApaI, AvrII, Pmel, SfiI, SacI, Kpnl, SmaI, BamHI, XbaI, SalI, PstI, SphI, HindIII, PacI, AscI Xhol* et *EcoNI.*

### C) Construction du vecteur pRP T:

- pRP O: un dérivé de pRPA-RD-153 contenant une cassette d'expression de l'HPPD, promoteur double histone - TEV - gène HPPD - terminateur Nos. Pour fabriquer pRP O, pRPA-RD153 est digéré par Hind III, traité par la Klenow puis redigéré par NcoI pour enlever le gène *oxy* et le remplacer par le gène HPPD sorti du plasmide pRP A par digestion BstEII, traitement par la Klenow et redigestion par NcoI.
- pRP R: pour l'obtenir le plasmide pRP O a été digéré par PvuII et SacI, le gène chimère a été purifié puis ligué dans pRPA-RD-185 lui-même digéré par PvuII et SacI.
- pRP T: il a été obtenu par ligation du gène chimère sorti de pRP R après digestion par SacI et HindIII dans le plasmide pRPA-BL 150 alpha2 digéré par les mêmes enzymes (Demande européennne EP n° 0 508 909).

Le gène chimère du vecteur pRP T a donc la structure suivante:

| | | | |
|---|---|---|---|
| Promoteur double histone | TEV | Région codante de l'HPPD | Terminateur nos |

### D) Construction du vecteur pRP V

- pRP P: c'est un dérivé de pRPA-RD-7 (Demande européennne EP n° 0 652 286) contenant le peptide de transit optimisé suivi du gène de l'HPPD. Il a été obtenu par ligation de la partie codante de l'HPPD sorti de pRP A par digestion BstEII et NcoI, traitement à la Klenow et du plasmide pRPA-RD-7 lui-même digéré SphI et AccI et traité à la DNAse polymérase T4.
- pRP Q: un dérivé de pRPA-RD-153 contenant une cassette d'expression de l'HPPD, promoteur double histone - TEV - OTP - gène HPPD - terminateur Nos. Pour le construire le plasmide pRPA-RD-153 est digéré par Sal I, traité par la Klenow puis redigéré par NcoI pour enlever le gène *oxy* et le remplacer par le gène HPPD sorti du plasmide pRP P par digestion BstEII, traitement par la Klenow et redigestion par NcoI.
- pRP S: pour l'obtenir, le plasmide pRP Q a été digéré par PvuII et SacI pour sortir le gène chimère qui a été ligué dans pRPA-RD-185 lui-même digéré par PvuII et SacI.
- pRP V: il a été obtenu par ligation du gène chimère sorti de pRP S après digestion par SacI et HindIII dans le plasmide pRPA-BL 150 alpha2 (Demande européennne EP n° 0 508 909).

Le gène chimère du vecteur pRP Q a donc la structure suivante:

| | | | | |
|---|---|---|---|---|
| Promoteur double histone | TEV | OTP | Région codante de l'HPPD | Terminateur nos |

### Exemple 3: Transformation du tabac industriel PBD6.

Afin de déterminer l'efficacité de ces deux gènes chimériques, ceux-ci ont été transférés dans du tabac industriel PBD6 selon les procédures de transformation et de régénération déjà décrites dans la demande européenne EP n° 0 508 909.

### 1)Transformation:

Le vecteur est introduit dans la souche non oncogène *d'Agrobacterium* EHA 101(Hood et al,1987) porteuse du cosmide pTVK 291(Komari et al,1986).La technique de transformation est basée sur la procédure de Horsh R. et al. (1985) Science, 227, 1229-1231.

### 2) Régénération:

La régénération du tabac PBD6 (provenance SETTA France) à partir d'explants foliaires est réalisée sur un milieu de base Murashige et Skoog (MS) comprenant 30g/l de saccharose ainsi que 200ug/ml de kanamycine.Les explants foliaires sont prélevés sur des plants en serre ou in vitro et transformés selon la technique des disques foliaires(Science 1985,Vol 227 ,p. 1229-1231) en trois étapes successives:la première comprend l'induction des pousses sur un milieu MS additionné de 30g/l de saccharose contenant 0.05mg/l d'acide naphtylacétique (ANA) et 2 mg/l de benzylaminopurine (BAP) pendant 15 jours. Les pousses formées au cours de cette étape sont ensuite développées par culture sur un milieu MS additionné de 30 g/l de saccharose mais ne contenant pas d'hormone, pendant 10 jours. Puis on prélève des pousses développées et on les cultive sur un milieu d'enracinement MS à teneur moitié en sels, vitamines et sucres et ne contenant pas d'hormone. Au bout d'environ 15 jours, les pousses enracinées sont passées en terre.

### Exemple 4: Mesure de la tolérance du tabac au 4-[4-CF3-2-(méthylsulfonyl) benzoyl]-5-cyclopropyl isoxazole: traitement de postlevée.

Au sortir de l'in-vitro, les plantules de tabac transformées ont été acclimatées à la serre (60% d'humidité relative; température: 20°C la nuit et 23°C la jour) pendant cinq semaines puis traitées au 4-[4-CF3-2-(méthylsulfonyl)benzoyl]-5-cyclopropyl isoxazole.

Le tabac témoin, non transformé et traité au 4-[4-CF3-2-(méthylsulfonyl)benzoyl]-5-cyclopropyl isoxazole à des doses allant de 50 à 400 g/ha, développe en environ 72 heures des chloroses, qui s'intensifient pour évoluer vers des nécroses très prononcées en une semaine( couvrant environ 80% des feuilles terminales).

Après transformation ce même tabac, qui surexprime l'HPPD de *P. fluorescens,* est très bien protégé contre un traitement au 4-[4-CF3-2-(méthylsulfonyl)benzoyl]-5-cyclopropyl isoxazole à la dose de 400 g/ha.

Si l'enzyme surexprimée est dans le cytoplasme, c'est à dire si la transformation a été faite avec le gène porté par le vecteur pRP T, alors la plante présente de très légères chloroses toutes localisées sur les feuilles intermédiaires.

Si l'enzyme surexprimée est dans le chloroplaste, c'est à dire si la transformation a été faite avec le gène porté par le verteur pRP V, alors la plante est parfaitement protégée, ne présente aucun symptôme.

### Exemple 5: Mesure de la tolérancedu tabac au 4-[4-CF3-2-(méthylsulfoyl) benzoyl]-5-cyclopropyl isoxazole: traitement de prélevée

### a) test in vitro:

On utilise des graines de tabac récoltées à partir des plantes issues du cycle "transformation - régénération" résistantes à un traitement foliaire d'isoxaflutole à la dose de 400g/h décrites aux exemples 1 à 3.

Ces graines ont été semées sur des boites contenant du phytagar à 10 g/l et de l'isoxaflutole à différentes concentrations allant de 0 à 1 mg/l. La germination a été faite ensuite à 25°C avec une photopériode de 12 heures de lumière/12 heures d'obscurité.

Selon ce protocole des graines de tabacs sauvages ont été mises à germer ainsi que des graines des deux types de tabacs transgéniques c'est à dire tabacs CY, avec localisation de l'HPPD dans le cytoplasme, et les tabacs CO avec localisation de l'HPPD dans le chloroplaste.

Les mesures de résistances sont faites visuellement entre 2 et 3 semaines après le semis.

Les résultats sont consignés dans le tableau ci-dessous.

| concentration en isoxaflutole | Tabac sauvage | Tabac CY | Tabac CO |
|---|---|---|---|
| 0 mg/l | 100% des graines germent sans symptômes° | 100% des graines germent sans symptômes' | 100% des graines germent sans symptômes |
| 0,05 mg/l | 20% des graines germent et présentent des symptômes° | 75% des graines germent* sans symptômes° | 75% des graines germent* sans symptômes° |
| 0,1 mg/l | pas de germination | 75% des graines germent* sans symptômes° | 75% des graines germent* sans symptômes° |
| 0,5 mg/l | pas de germination | 75% des graines germent* sans symptômes° | 75% des graines germent* sans symptômes° |
| 1 mg/l | pas de germination | 75% des graines germent* avec légers symptômes° | 75% des graines germent* sans symptômes° |

| | | | |
|---|---|---|---|
| ° les symptômes que présentent les plantules en cours de germination sont des déformations des cotylédons plus ou moins importantes et surtout un blanchiment des tissus normalement photosynthétiques et donc verts. * 75% des graines germent car ont été semées des graines issues de l'autofécondation de plantes mono-loccus sortant du cycle "transformation - régénération" ne portant donc le gène de toléranceque sur un chromosome. | | | |

En opérant de la même manière avec les produits suivants produit n° 51 du brevet américain 4 780 127, on obtient les mêmes résultats à une concentration de 0 mg/l et 0,1 mg/l sur tabac sauvage et tabac CO.

### b) test en serre:

On opère comme à l'exemple 4, si ce n'est que le traitement est effectué en prélevée, 24 heures avant le semis. Le semis sauvage s'effectue normalement. Dans ces conditions on observe que pour les semis témoins non traités, il n'y pas de germination pour toute dose d'herbicide au moins égale à 10 g/ha. Au contraire les tabacs CY ne présentent aucun symptôme, tel que défini au paragraphe a), jusqu'à 100 g/ha compris. De même les tabacs CO ne présentent aucun symptôme, tel que défini au paragraphe a) jusqu'à 200 g/ha compris.

Ces résultats montrent clairement que le gène de l'HPPD de *P. fluorescens* confère une toléranceau tabac contre les traitements en prélevée à l'isoxaflutole. Cette toléranceest meilleure si la protéine est localisée dans le chloroplaste au lieu du cytoplasme.

### Exemple 6:

Dans le but d'étudier si le gène de l'HPPD de *Pseudomonas fluorescens* peut être utilisé comme gène marqueur au cours du cycle "transformation - régénération" d'une espèce végétale, le tabac a été transformé avec le gène de l'HPPD et des plantes transformées ont été obtenues après sélection sur isoxaflutole.

### Matériel et méthodes et résultats

Le gène chimérique pRP V décrit ci-dessous est transféré dans du tabac industriel PBD6 selon les procédures de transformation et de régénération déjà décrites dans la demande européenne EP n° 0 508 909.

Le gène chimère du vecteur pRP V a la structure suivante:

| | | | | |
|---|---|---|---|---|
| Promoteur double histone | TEV | OTP | Région codante de l'HPPD | Terminateur nos |

### 1)Transformation:

Le vecteur est introduit dans la souche non oncogène *d'Agrobacterium* EHA 101 (Hood et al, 1987) porteuse du cosmide pTVK 291 (Komari et al, 1986). La technique de transformation est basée sur la procédure de Horsh et al(1985).

### 2) Régénération:

La régénération du tabac PBD6 (provenance SEITA France) à partir d'explants foliaires est réalisée sur un milieu de base Murashige et Skoog (MS) comprenant 30g/l de saccharose ainsi que 350 mg/l de cefotaxime et 1 mg/l d'isoxaflutole. Les explants foliaires sont prélevés sur des plants en serre ou in vitro et transformés selon la technique des disques foliaires(Science 1985,Vol 227,p. 1229-1231) en trois étapes successives: la première comprend l'induction des pousses sur un milieu MS additionné de 30g/l de saccharose contenant 0.05mg/l d'acide naphtylacétique (ANA) et 2 mg/l de benzylaminopurine (BAP) pendant 15 jours et 1 mg/l d'isoxaflutole. Les pousses vertes formées au cours de cette étape sont ensuite développées par culture sur un milieu MS additionné de 30 g/l de saccharose et 1 mg/l d'isoxaflutole mais ne contenant pas d'hormone, pendant 10 jours. Puis on prélève des pousses développées et on les cultive sur un milieu d'enracinement MS à teneur moitié en sels, vitamines et sucres et 1 mg/l d'isoxaflutole et ne contenant pas d'hormone. Au bout d'environ 15 jours, les pousses enracinées sont passées en terre.

Toutes les plantules obtenues selon ce protocole sont analysées par PCR avec des amorces spécifiques de l'HPPD de *P.fluorescens.* Cette analyse PCR a permis de confirmer que toutes les plantules ainsi obtenues ont bien intégré le gène de l'HPPD.

En conclusion, cet essai confirme que le gène de l'HPPD peut être utilisé comme gène marqueur et que, associé à ce gène, l'isoxaflutole peut être un bon agent de sélection.

Exemples 7 et 8 : Isolement du gène de l'HPPD *d'Arabidopsis thaliana* et du gène de l'HPPD de carotte *(Daucus carotta)*
a) Construction des banques d'ADNc.
   Des mRNAs extraits de jeunes plantules *d'Arabidopsis thaliana,* et des mRNAs extraits de cellules de carotte en culture, ont servi à construire deux banques d'ADNc dans le vecteur Uni Zap™ XR commercialisé par la société Stratagen, suivant le protocole préconisé par cette société.
b) Criblage des banques d'ADNc
   Ces deux banques ont été criblées à l'aide d'une sonde correspondant à un ADNc *d'Arabidopsis thaliana* de longueur partielle, obtenu via l'Arabidopsis Biological Resource Center (Ohio, USA) et répertorié : EST clone N° 91B13T7. Ce clone est constitué d'environ 500 paires de bases dont seulement 228 avaient été séquencées par le MSU-DOE Plant Research Laboratory dans le cadre du séquençage au hasard des ADNc *d'Arabidopsis thaliana.* Nous avons séquencé complètement les 500 paires de bases avant d'utiliser ce clone pour cribler nos banques d'ADNc *d'Arabidopsis thaliana* et de carotte à l'aide de la technique classique d'hybridation des plages de lyse (*référence ?*).
c) Un ADNc *d'Arabidopsis thaliana* (SEQ ID N° 2) correspondant à 1338 paires de bases a été obtenu. Cet ADNc possède un codon de début d'initiation de la traduction à la position 25 et un codon de fin de traduction à la position 1336. Cet ADNc est donc complet et code pour une protéine de 445 acides aminés.
d) Un ADNc de carotte *(Daucus carotta)* (SEQ ID N° 3) correspondant à 1329 paires de bases à été obtenu. Cet ADNc possède un codon de début d'initiation de la traduction à la position 1 et un codon de fin de traduction à la position 1329. Cet ADNc est donc complet et code pour une protéine de 442 acides aminés.

Les séquences illustrées sont les suivantes:
SEQ ID N° 1 Séquence du gène de l'HPPD de *Pseudomonas fluorescens* A32.
SEQ ID N° 2
   Séquence d' ADNc d'HPPD *d'Arabidopsis thaliana*
SEQ ID N° 3
   séquence d' ADNc d'HPPD de *Daucus carotta*

Les figures ci-après sont données à titre indicatif pour illustrer l'invention.
La Figure 1 représente la séquence protéique de l'HPPD de *Pseudomonas sp.* strain P.J. 874 et la séquence nucléotidique théorique de la partie codante correspondante; les cinq oligonucléotides choisis pour faire l'amplification d'une partie de cette région codante sont symbolisés par les cinq fléches.
La Figure 2 représente la cartographie du plasmide avec le fragment d'ADN génomique de 7 kb contenant le gène de l'HPPD de *P. fluorescens* A32.
La Figure 3 donne la comparaison des séquences en acides aminés de l' HPPD de *P*. *fluorescens A32* et de l'HPPD de *Pseudomonas sp* strain P.J. 874 (seuls les acides aminés divergents entre les deux séquences sont indiqués) ainsi que la séquence consensus.

## Revendications

1. Gène chimère comprenant dans le sens de la transcription:
- au moins une séquence de régulation promotrice issue d'un gène s'exprimant naturellement dans les plantes,
- une séquence de peptide de transit d'un gène végétal codant pour une enzyme à localisation plastidiale entre la séquence de régulation promotrice et la séquence codante,
- une séquence codante hétérologue,
- au moins une séquence de régulation terminatrice ou de polyadénylation,
**caractérisé en ce que** la séquence codante hétérologue est la séquence codante d'un gène qui exprime une hydroxy-phényl pyruvate dioxygénase (HPPD).

2. Gène chimère selon la revendication 1, **caractérisé en ce que** la séquence codante est d'origine bactérienne ou de plante.

3. Gène chimère selon la revendication 2, **caractérisé en ce que** la séquence codante est issue de *Pseudomonas sp.*

4. Gène chimère selon la revendication 3, **caractérisée en ce que** la séquence codante est issue de *Pseudomonas fluorescens.*

5. Gène chimère selon la revendication 4, **caractérisée en ce que** la séquence codante est représentée par la SEQ ID NO 1.

6. Gène chimère selon la revendication 2, **caractérisée en ce** la séquence codante est d'origine végétale.

7. Gène chimère selon la revendication 6, **caractérisée en ce que** la séquence codante est issue d*'Arabidopsis.*

8. Gène chimère selon la revendication 7, **caractérisée en ce que** la séquence codante est représentée par la SEQ ID NO 2.

9. Gène chimère selon la revendication 6, **caractérisée en ce que** la séquence codante est issue d'une ombellifère.

10. Gène chimère selon la revendication 9, **caractérisée en ce que** la séquence codante est issue de la carotte *(Daucus carotta).*

11. Gène chimère selon la revendication 10, **caractérisée en ce que** la séquence codante est représentée par la SEQ ID NO 3.

12. Gène chimère selon l'une des revendications 1 à 11, **caractérisée en ce que** la séquence de régulation promotrice favorise la surexpression de la séquence codante.

13. Gène chimère selon la revendication 12, **caractérisé en ce que** la séquence de régulation promotrice comprend au moins un promoteur d'histone.

14. Gène chimère selon l'une des revendications 1 à 13, **caractérisé en ce qu'**il comprend, entre la séquence de régulation promotrice et la séquence codante, un peptide de transit optimisé comprenant, dans le sens de la transcription, une séquence codant pour un peptide de transit d'un gène végétal codant pour une enzyme à localisation plastidiale, une partie de séquence de la partie mature N terminale d'un gène végétal codant pour une enzyme à localisation plastidiale, puis une séquence codant pour un second peptide de transit d'un gène végétal codant pour une enzyme à localisation plastidiale.

15. Gène chimère selon l'une des revendications 1 à 14, **caractérisé en ce qu'**il comprend une séquence d'un activateur de transcription (enhancer) entre la séquence de régulation promotrice et la séquence codante.

16. Vecteur utilisable pour la transformation génétique des plantes, **caractérisé en ce qu'**il comprend un gène chimère selon l'une des revendications 1 à 15.

17. Cellule végétale, **caractérisée en ce qu'**elle comprend un gène chimère selon l'une des revendications 1 à 15.

18. Plante, **caractérisée en ce qu'**elle est régénérée à partir de cellules selon la revendication 17.

19. Plante, **caractérisée en ce qu'**elle comprend dans son génome une quantité efficace d'un gène chimère selon l'une des revendications 1 à 15.

20. Plante selon l'une des revendications 18 ou 19, **caractérisée en ce qu'**elle appartient à la famille des dicotylédones.

21. Plante selon l'une des revendications 18 ou 19, **caractérisée en ce qu'**elle appartient à la famille des monocotylédones.

22. Procédé de transformation de plantes pour les rendre tolérantes aux inhibiteurs d'HPPD, **caractérisé en ce qu'**on introduit dans une cellule végétale un gène chimère selon l'une des revendications 1 à 15.

23. Procédé selon la revendication 22, **caractérisé en ce que** le transfert est effectué avec *Agrobacterium tumefaciens* ou *Agrobacterium rhizogenes.*

24. Procédé selon la revendication 22, **caractérisé en ce que** le transfert est effectué par apport par bombardement à l'aide de particules chargées de l'ADN.

25. Procédé de transformation de plantes, **caractérisé en ce qu'**on introduit dans la cellule végétale un gène selon l'une des revendications 1 à 15 comme marqueur de sélection.

26. Procédé de traitement herbicide sélectif de plantes, **caractérisé en ce qu'**on applique un inhibiteur d'HPPD à une plante transformée comprenant des cellules selon la revendication 17.

27. Procédé selon la revendication 26, **caractérisé en ce que** les plantes sont les plantes selon les revendications 18 à 21.

28. Procédé selon l'une des revendications 26 ou 27, **caractérisé en ce que** les plantes sont obtenues par le procédé selon les revendications 22 à 24.

29. Procédé selon l'une des revendications 26 à 28, **caractérisé en ce que** l'inhibiteur d'HPPD est un isoxazole, un dicétonitrile, une tricétone ou la sulcotrione.

30. Procédé selon la revendication 29, **caractérisé en ce que** l'isoxazole est le 4-[4-CF3-2-(méthylsulfoyl)benzoyl]-5-cyclopropyl isoxazole.

## Claims

1. Chimeric gene comprising, in the transcription direction:
- at least one promoter regulation sequence from a gene expressing itself naturally in plants,
- a transit peptide sequence of a plant gene coding for a plastid localization enzyme between the promoter regulation sequence and the coding sequence,
- a heterologous coding sequence,
- at least one terminator regulation or polyadenylation sequence,
**characterized in that** the heterologous coding sequence is the coding sequence of a gene which expresses a hydroxyphenylpyruvate dioxygenase (HPPD).

2. Chimeric gene according to Claim 1, **characterized in that** the coding sequence is of bacterial or plant origin.

3. Chimeric gene according to Claim 2, **characterized in that** the coding sequence is from *Pseudomonas sp.*

4. Chimeric gene according to Claim 3, **characterized in that** the coding sequence is from *Pseudomonas fluorescens.*

5. Chimeric gene according to Claim 4, **characterized in that** the coding sequence is represented by SEQ ID No. 1.

6. Chimeric gene according to Claim 2, **characterized in that** the coding sequence is of plant origin.

7. Chimeric gene according to Claim 6, **characterized in that** the coding sequence is from *Arabidopsis.*

8. Chimeric gene according to Claim 7, **characterized in that** the coding sequence is represented by SEQ ID No. 2.

9. Chimeric gene according to Claim 6, **characterized in that** the coding sequence is from an Umbellifera.

10. Chimeric gene according to Claim 9, **characterized in that** the coding sequence is from carrot (*Daucus carota*)*.*

11. Chimeric gene according to Claim 10, **characterized in that** the coding sequence is represented by SEQ ID No. 3.

12. Chimeric gene according to one of Claims 1 to 11, **characterized in that** the promoter regulation sequence favours the overexpression of the coding sequence.

13. Chimeric gene according to Claim 12, **characterized in that** the promoter regulation sequence comprises at least one histone promoter.

14. Chimeric gene according to one of Claims 1 to 13, **characterized in that** it comprises, between the promoter regulation sequence and the coding sequence, an optimized transit peptide comprising, in the transcription direction, a sequence coding for a transit peptide of a plant gene coding for a plastid localization enzyme, a part sequence of the N-terminal mature part of a plant gene coding for a plastid localization enzyme, then a sequence coding for a second transit peptide of a plant gene coding for a plastid localization enzyme.

15. Chimeric gene according to one of Claims 1 to 14, **characterized in that** it comprises a sequence of a transcription activator (enhancer) between the promoter regulation sequence and the coding sequence.

16. Vector utilizable for the genetic transformation of plants, **characterized in that** it comprises a chimeric gene according to one of Claims 1 to 15.

17. Plant cell, **characterized in that** it comprises a chimeric gene according to one of Claims 1 to 15.

18. Plant, **characterized in that** it is regenerated from cells according to Claim 17.

19. Plant, **characterized in that** it comprises, in its genome, an effective quantity of a chimeric gene according to one of Claims 1 to 15.

20. Plant according to either of Claims 18 and 19, **characterized in that** it belongs to the dicotyledon family.

21. Plant according to either of Claims 18 and 19, **characterized in that** it belongs to the monocotyledon family.

22. Method of transformation of plants to make them tolerant to HPPD inhibitors, **characterized in that** a chimeric gene according to one of Claims 1 to 15 is introduced into a plant cell.

23. Method according to Claim 22, **characterized in that** the transfer is accomplished with *Agrobacterium tumefaciens* or *Agrobacterium rhizogenes.*

24. Method according to Claim 22, **characterized in that** the transfer is accomplished by addition by bombardment with the aid of particles loaded with the DNA.

25. Method of transformation of plants, **characterized in that** a gene according to one of Claims 1 to 15 is introduced into the plant cell as selection marker.

26. Method of selective herbicidal treatment of plants, **characterized in that** an HPPD inhibitor is applied to a transformed plant comprising cells according to Claim 17.

27. Method according to Claim 26, **characterized in that** the plants are the plants according to Claims 18 to 21.

28. Method according to either of Claims 26 and 27, **characterized in that** the plants are obtained by the method according to Claims 22 to 24.

29. Method according to one of Claims 26 to 28, **characterized in that** the HPPD inhibitor is an isoxazole, a diketonitrile, a triketone or sulcotrione.

30. Method according to Claim 29, **characterized in that** the isoxazole is 4-[4-CF3-2-(methylsulphoyl)benzoyl]-5-cyclopropyl isoxazole.

## Patentansprüche

1. Chimäres Gen, das in Transkriptionsrichtung folgendes enthält:
- mindestens eine Promotorregulationssequenz aus einem Gen, das auf natürliche Weise in Pflanzen exprimiert wird,
- eine Leitpeptidsequenz eines pflanzlichen Gens, das für ein Enzym mit Lokalisation im Plastiden codiert, zwischen der Promotorregulationssequenz und der Codiersequenz,
- eine heterologe Codiersequenz,
- mindestens eine Terminationsregulationssequenz oder Polyadenylierungssequenz,
**dadurch gekennzeichnet, daß** es sich bei der heterologen Codiersequenz um die Codiersequenz eines Gens handelt, das eine Hydroxyphenylpyruvat-Dioxygenase (HPPD) exprimiert.

2. Chimäres Gen nach Anspruch 1, **dadurch gekennzeichnet, daß** die Codiersequenz bakteriellen oder pflanzlichen Ursprungs ist.

3. Chimäres Gen nach Anspruch 2, **dadurch gekennzeichnet, daß** die Codiersequenz von *Pseudomonas sp* stammt.

4. Chimäres Gen nach Anspruch 3, **dadurch gekennzeichnet, daß** die Codiersequenz von *Pseudomonas fluorescens* stammt.

5. Chimäres Gen nach Anspruch 4, **dadurch gekennzeichnet, daß** die Codiersequenz der SEQ ID NO 1 entspricht.

6. Chimäres Gen nach Anspruch 2, **dadurch gekennzeichnet, daß** die Codiersequenz pflanzlichen Ursprungs ist.

7. Chimäres Gen nach Anspruch 6, **dadurch gekennzeichnet, daß** die Codiersequenz aus *Arabidopsis* stammt.

8. Chimäres Gen nach Anspruch 7, **dadurch gekennzeichnet, daß** die Codiersequenz der SEQ ID NO 2 entspricht.

9. Chimäres Gen nach Anspruch 6, **dadurch gekennzeichnet, daß** die Codiersequenz aus einer Umbellifere stammt.

10. Chimäres Gen nach Anspruch 9, **dadurch gekennzeichnet, daß** die Codiersequenz aus der Karotte *(Daucus carotta)* stammt.

11. Chimäres Gen nach Anspruch 10, **dadurch gekennzeichnet, daß** die Codiersequenz der SEQ ID NO 3 entspricht.

12. Chimäres Gen nach einem der Ansprüche 1 bis 11, **dadurch gekennzeichnet, daß** die Promotorregulationssequenz die Überexpression der Codiersequenz begünstigt.

13. Chimäres Gen nach Anspruch 12, **dadurch gekennzeichnet, daß** die Promotorregulationssequenz mindestens einen Histon-Promotor enthält.

14. Chimäres Gen nach einem der Ansprüche 1 bis 13, **dadurch gekennzeichnet, daß** es zwischen der Promotorregulationssequenz und der Codiersequenz ein optimiertes Leitpeptid enthält, das in Transkriptionsrichtung eine Codiersequenz für ein Leitpeptid eines pflanzlichen Gens, das für ein Enzym für Lokalisierung in den Plastiden codiert, eine Teilsequenz des reifen N-terminalen Abschnitts eines pflanzlichen Gens, das für ein Enzym für Lokalisierung in den Plastiden codiert, sowie eine Codiersequenz für ein zweites Leitpeptid eines pflanzlichen Gens, das für ein Enzym für Lokalisierung in den Plastiden codiert, enthält.

15. Chimäres Gen nach einem der Ansprüche 1 bis 14, **dadurch gekennzeichnet, daß** es zwischen der Promotorregulationsequenz und der Codiersequenz eine Sequenz eines Transkriptionsaktivators (Enhancers) enthält.

16. Vektor, der sich für die genetische Transformation von Pflanzen eignet, **dadurch gekennzeichnet, daß** er ein chimäres Gen nach einem der Ansprüche 1 bis 15 enthält.

17. Pflanzliche Zelle, **dadurch gekennzeichnet, daß** sie ein chimäres Gen nach einem der Ansprüchen 1 bis 15 enthält.

18. Pflanze, **dadurch gekennzeichnet, daß** sie aus Zellen nach Anspruch 17 regeneriert wird.

19. Pflanze, **dadurch gekennzeichnet, daß** sie in ihrem Genom eine wirksame Menge eines chimären Gens nach einem der Ansprüche 1 bis 15 enthält.

20. Pflanze nach Anspruch 18 oder 19, **dadurch gekennzeichnet, daß** sie zur Familie der dicotylen Pflanzen gehört.

21. Pflanze nach Anspruch 18 oder 19, **dadurch gekennzeichnet, daß** sie zur Familie der monocotylen Pflanzen gehört.

22. Verfahren zur Transformation von Pflanzen, um diese gegenüber HPPD-Hemmern tolerant zu machen, **dadurch gekennzeichnet, daß** man ein chimäres Gen nach einem der Ansprüche 1 bis 15 in einer Pflanzenzelle einbringt.

23. Verfahren nach Anspruch 22, **dadurch gekennzeichnet, daß** der Transfer mit *Agrobacterium tumefaciens* oder *Agrobacterium rhizogenes* erfolgt.

24. Verfahren nach Anspruch 22, **dadurch gekennzeichnet, daß** der Transfer durch Beschuß mit DNA-haltigen Teilchen erfolgt.

25. Verfahren zur Transformation von Pflanzen, **dadurch gekennzeichnet, daß** man ein Gen nach einem der Ansprüche 1 bis 15 als Selektionsmarker in die pflanzliche Zelle einbringt.

26. Verfahren zur selektiven Herbizidbehandlung von Pflanzen, **dadurch gekennzeichnet, daß** man auf eine transformierte Pflanze, die Zellen nach Anspruch 17 enthält, einen HPPD-Hemmer ausbringt.

27. Verfahren nach Anspruch 26, **dadurch gekennzeichnet, daß** es sich bei den Pflanzen um die Pflanzen nach den Ansprüchen 18 bis 21 handelt.

28. Verfahren nach Anspruch 26 oder 27, **dadurch gekennzeichnet, daß** die Pflanzen nach dem Verfahren nach den Ansprüchen 22 bis 24 erhalten werden.

29. Verfahren nach einem der Ansprüche 26 bis 28, **dadurch gekennzeichnet, daß** es sich bei dem HPPD-Hemmer um ein Isoxazol, ein Diketonitril, ein Triketon oder um Sulcotrion handelt.

30. Verfahren nach Anspruch 29 **dadurch gekennzeichnet, daß** es sich bei dem Isoxazol um 4-[4-CF₃-2-(Methylsulfoyl)benzoyl]-5-cyclopropylisoxazol handelt.


### Liste de séquences

(1) GENERAL INFORMATION:
   (i) APPLICANT: Sailland, Alain
      Rolland, Anne
      Matringe, Michel
      Pallett, Kenneth E
   (ii) TITLE OF INVENTION: SEQUENCE ADN D'UN GENE DE L'HYDROXY-PHENYL PYRUVATE DIOXYGENASE ET OBTENTION DE PLANTES CONTENANT CE GENE DE L'HYDROXY-PHENYL PYRUVATE DIOXYGENASE, RESISTANTES A CERTAINS HERBICIDES
   (iii) NUMBER OF SEQUENCES: 3
   (iv) CORRESPONDENCE ADDRESS:
      (A) ADDRESSEE: Francois Chretien
      (B) STREET: 14-20 rue Pierre BAIZET
      (C) CITY: Lyon Cedex 09
      (E) COUNTRY: France
      (F) ZIP: 69263
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #125
   (vi) CURRENT APPLICATION DATA:
      (A) APPLICATION NUMBER: FR PH95033
      (B) FILING DATE: 02-JUN-1995
      (C) CLASSIFICATION:
   (viii) ATTORNEY/AGENT INFORMATION:
      (A) NAME: Chrétien, Francois
   (ix) TELECOMMUNICATION INFORMATION:
      (A) TELEPHONE: 72-29-26-42
      (B) TELEFAX: 72-29-28-43
(2) INFORMATION FOR SEQ ID NO:1:
   (i) SEQUENCE CHARACTERISTICS:
      (A) LENGTH: 1077 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Pseudomonas fluorescens
   (xi) SEQUENCE DESCRIPTION: SEQ ID NO:1:
   ATGGCAGATC TATACGAAAA CCCAATGGGC CTGATGGGCT TTGAATTCAT CGAATTAGCG      60
   TCGTTGACGC CGGGTACCCT GGAGCCGATC TTCGAGATCA TGGGCTTCAC CAAAGTCGCG     120
   ACCCACCGTT CCAAGAACGT GCACCTGTAC CGCCAGGGCG AGATCAACCT GATCCTCAAC     180
   AACGAGCCCA ACAGCATCGC CTCCTACTTT GCGGCCGAAC ACGGCCCGTC GGTGTGCGGC     240
   ATGGCGTTCC GCGTGAAGGA CTCGCAAAAG GCCTACAACC GCGCCCTGGA ACTCGGCGCC     300
   CAGCCGATCC ATATTGACAC CGGGCCGATG GAATTGAACC TGCCGGCGAT CAAGGGCATC     360
   GGCGGCGCGC CGTTGTACCT GATCGACCGT TTCGGCGAAG GCAGCTCGAT CTACGACATC     420
   GACTTCGTGT ACCTCGAAGG TGTGGAGCGC AATCCGGTCG GTGCAGGTCT CAAAGTCATC     480
   GACCACCTGA CCCACAACGT CTATCGCGGC CGCATGGTCT ACTGGGCCAA CTTCTACGAG     540
   AAATTGTTCA ACTTCCGTGA AGCGCGTTAC TTCGATATCA AGGGCGAGTA CACCGGCCTG     600
   ACTTCCAAGG CCATGAGTGC GCCGGACGGC ATGATCCGCA TCCCGCTGAA CGAAGAGTCG     660
   TCCAAGGGCG CGGGGCAGAT CGAAGAGTTC CTGATGCAGT TCAACGGCGA AGGCATCCAG     720
   CACGTGGCGT TCCTCACCGA CGACCTGGTC AAGACCTGGG ACGCGTTGAA GAAAATCGGC     780
   ATGCGCTTCA TGACCGCGCC GCCAGACACT TATTACGAAA TGCTCGAAGG CCGCCTGCCT     840
   GACCACGGCG AGCCGGTGGA TCAACTGCAG GCACGCGGTA TCCTGCTGGA CGGATCTTCC     900
   GTGGAAGGCG ACAAACGCCT GCTGCTGCAG ATCTTCTCGG AAACCCTGAT GGGCCCGGTG     960
   TTCTTCGAAT TCATCCAGCG CAAGGGCGAC GATGGGTTTG GCGAGGGCAA CTTCAAGGCG    1020
   CTGTTCGAGT CCATCGAACG TGACCAGGTG CGTCGTGGTG TATTGACCGC CGATTAA       1077
(2) INFORMATION FOR SEQ ID NO:2:
   (i) SEQUENCE CHARACTERISTICS:
      (A) LENGTH: 1338 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (v) ORIGINAL SOURCE:
      (A) ORGANISM: Arabidopsis thaliana
      (B) STRAIN: Columbia
      (D) DEVELOPMENTAL STAGE: Young green plant
   (vi) IMMEDIATE SOURCE:
      (A) LIBRARY: Uni zap XR STRATAGENE
   (xi) SEQUENCE DESCRIPTION: SEQ ID NO:2:
   ATGGGCCACC AAAACGCCGC CGTTTCAGAG AATCAAAACC ATGATGACGG CGCTGCGTCG     60
   TCGCCGGGAT TCAAGCTCGT CGGATTTTCC AAGTTCGTAA GAAAGAATCC AAAGTCTGAT    120
   AAATTCAAGG TTAAGCGCTT CCATCACATC GAGTTCTGGT GCGGCGACGC AACCAACGTC    180
   GCTCGTCGCT TCTCCTGGGG TCTGGGGATG AGATTCTCCG CCAAATCCGA TCTTTCCACC    240
   GGAAACATGG TTCACGCCTC TTACCTACTC ACCTCCGGTG ACCTCCGATT CCTTTTCACT    300
   GCTCCTTACT CTCCGTCTCT CTCCGCCGGA GAGATTAAAC CGACAACCAC AGCTTCTATC    360
   CCAAGTTTCG ATCACGGCTC TTGTCGTTCC TTCTTCTCTT CACATGGTCT CGGTGTTAGA    420
   GCCGTTGCGA TTGAAGTAGA AGACGCAGAG TCAGCTTTCT CCATCAGTGT AGCTAATGGC    480
   GCTATTCCTT CGTCGCCTCC TATCGTCCTC AATGAAGCAG TTACGATCGC TGAGGTTAAA    540
   CTATACGGCG ATGTTGTTCT CCGATATGTT AGTTACAAAG CAGAAGATAC CGAAAAATCC    600
   GAATTCTTGC CAGGGTTCGA GCGTGTAGAG GATGCGTCGT CGTTCCCATT GGATTATGGT    660
   ATCCGGCGGC TTGACCACGC CGTGGGAAAC GTTCCTGAGC TTGGTCCGGC TTTAACTTAT    720
   GTAGCGGGGT TCACTGGTTT TCACCAATTC GCAGAGTTCA CAGCAGACGA CGTTGGAACC    780
   GCCGAGAGCG GTTTAAATTC AGCGGTCCTG GCTAGCAATG ATGAAATGGT TCTTCTACCG    840
   ATTAACGAGC CAGTGCACGG AACAAAGAGG AAGAGTCAGA TTCAGACGTA TTTGGAACAT    900
   AACGAAGGCG CAGGGCTACA ACATCTGGCT CTGATGAGTG AAGACATATT CAGGACCCTG    960
   AGAGAGATGA GGAAGAGGAG CAGTATTGGA GGATTCGACT TCATGCCTTC TCCTCCGCCT   1020
   ACTTACTACC AGAATCTCAA GAAACGGGTC GGCGACGTGC TCAGCGATGA TCAGATCAAG   1080
   GAGTGTGAGG AATTAGGGAT TCTTGTAGAC AGAGATGATC AAGGGACGTT GCTTCAAATC   1140
   TTCACAAAAC CACTAGGTGA CAGGCCGACG ATATTTATAG AGATAATCCA GAGAGTAGGA   1200
   TGCATGATGA AAGATGAGGA AGGGAAGGCT TACCAGAGTG GAGGATGTGG TGGTTTTGGC   1260
   AAAGGCAATT TCTCTGAGCT CTTCAAGTCC ATTGAAGAAT ACGAAAAGAC TCTTGAAGCC   1320
   AAACAGTTAG TGGGATGA                                                 1338
(2) INFORMATION FOR SEQ ID NO:3:
   (i) SEQUENCE CHARACTERISTICS:
      (A) LENGTH: 1329 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Daucus carota
      (D) DEVELOPMENTAL STAGE: Suspension cells
   (vii) IMMEDIATE SOURCE:
      (A) LIBRARY: Uni zap XR STRATAGENE
   (xi) SEQUENCE DESCRIPTION: SEQ ID NO:3:
   ATGGGGAAAA AACAATCGGA AGCTGAAATT CTCTCAAGCA ATTCATCAAA CACCTCTCCT     60
   GCAACATTCA AGCTGGTCGG TTTCAACAAC TTCGTCCGCG CCAACCCCAA GTCCGATCAC    120
   TTCGCCGTGA AGCGGTTCCA CCACATTGAG TTCTGGTGCG GCGACGCCAC CAACACGTCG    180
   CGGCGGTTCT CGTGGGGCCT CGGCATGCCT TTGGTGGCGA AATCGGATCT CTCTACTGGA    240
   AACTCTGTTC ACGCTTCTTA TCTTGTTCGC TCGGCGAATC TCAGTTTCGT CTTCACCGCT    300
   CCTTACTCTC CGTCCACGAC CACTTCCTCT GGTTCAGCTG CCATCCCGTC TTTTTCGGCA    360
   TCGGGTTTTC ACTCTTTTGC GGCCAAACAC GGCCTTGCTG TTCGGGCTAT TGCTCTTGAA    420
   GTTGCTGACG TGGCTGCTGC GTTTGAGGCC AGTGTTGCGC GTGGGGCCAG GCCGGCTTCG    480
   GCTCCTGTTG AATTGGACGA CCAGGCGTGG TTGGCTGAGG TGGAGTTGTA CGGAGATGTG    540
   GTCTTGAGGT TTGTTAGTTT TGGGAGGGAG GAGGGTTTGT TTTTGCCTGG ATTCGAGGCG    600
   GTGGAGGGGA CGGCGTCGTT TCCGGATTTG GATTATGGAA TTAGAAGACT TGATCATGCG    660
   GTGGGGAATG TTACCGAGTT GGGGCCTGTG GTGGAGTATA TTAAAGGGTT TACGGGGTTT    720
   CATGAATTTG CGGAGTTTAC AGCGGAGGAT GTGGGGACTT TGGAGAGTGG GTTGAATTCG    780
   GTGGTGTTGG CGAATAATGA GGAGATGGTT CTGTTGCCCT TGAATGAGCC TGTGTATGGG    840
   ACCAAGAGGA AGAGTCAGAT ACAGACTTAC TTGGAGCACA ATGAAGGGGC TGGAGTGCAG    900
   CATTTGGCTT TAGTGAGTGA GGATATTTTT AGGACTTTAA GGGAGATGAG GAAGAGGAGT    960
   TGCCTTGGTG GTTTTGAGTT TATGCCTTCG CCACCGCCTA CGTATTACAA GAATTTGAAG   1020
   AATAGGGTCG GGGATGTGTT GAGTGATGAA CAGATCAAGG AGTGTGAAGA TTTGGGGATT   1080
   TTGGTGGATA GGGATGATCA GGGTACATTG CTTCAAATCT TTACCAAGCC TGTAGGTGAC   1140
   AGGCCTACCT TATTCATAGA GATCATTCAG AGGGTAGGGT GCATGCTCAA GGACGATGCA   1200
   GGGCAGATGT ACCAGAAGGG CGGGTGCGGA GGATTTGGGA AGGGGAACTT CTCAGAGCTG   1260
   TTCAAGTCCA TCGAAGAATA TGAAAAAACA CTTGAAGCTA AACAAATCAC TGGATCTGCT   1320
   GCTGCATGA                                                           1329